(19) Europäisches Patentamt
European Patent Office
Office européen des brevets

(11) EP 4 394 366 A1

(12) **EUROPEAN PATENT APPLICATION**
published in accordance with Art. 153(4) EPC

(43) Date of publication:
**03.07.2024 Bulletin 2024/27**

(21) Application number: **22861335.2**

(22) Date of filing: **22.08.2022**

(51) International Patent Classification (IPC):
***G01N 24/08*** *(2006.01)*

(52) Cooperative Patent Classification (CPC):
**G01N 24/08**

(86) International application number:
**PCT/JP2022/031633**

(87) International publication number:
**WO 2023/027047 (02.03.2023 Gazette 2023/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.08.2021 JP 2021135920**

(71) Applicant: **OSAKA UNIVERSITY**
**Suita-shi**
**Osaka 565-0871 (JP)**

(72) Inventors:
- **MATSUKI, Yoh**
**Suita-shi, Osaka 565-0871 (JP)**
- **KATO, Ken**
**Suita-shi, Osaka 565-0871 (JP)**
- **FUJIWARA, Toshimichi**
**Suita-shi, Osaka 565-0871 (JP)**

(74) Representative: **Vossius & Partner Patentanwälte Rechtsanwälte mbB**
**Siebertstrasse 3**
**81675 München (DE)**

(54) **DNP-NMR POLARIZING AGENT, METHOD FOR MANUFACTURING SAME, AND METHOD FOR USING SAME**

(57) Provided are a polarizing agent that can selectively enhance NMR signals by the DNP-NMR method even in a reducing environment, and a method for selectively enhancing NMR signals. The present invention is a polarizing agent for use in the DNP-NMR method, the polarizing agent comprising nanodiamond particles, and the nanodiamond particles having a surface modified with a functional group capable of covalently binding to or interacting with proteins. The polarizing agent of the present invention can selectively enhance NMR signals by the DNP-NMR method even in a reducing environment.

Fig. 2

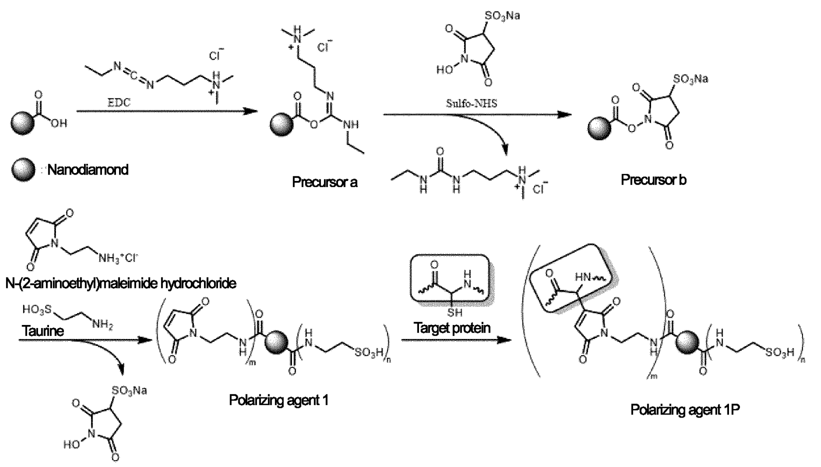


EP 4 394 366 A1

## Description

### Technical Field

The present invention relates to a polarizing agent for use in the dynamic nuclear polarization (DNP)-nuclear magnetic resonance (NMR) method (DNP-NMR method), a method for producing the same, and a method for using the same.

### Background Art

The DNP-NMR method is known as a technique for increasing NMR sensitivity because it makes it possible to enhance NMR signals by transferring the thermal polarization of electron spins, which is 660 times larger than the thermal polarization of nuclear spins, to the nuclear spins. In the DNP-NMR method, a radical polarizing agent, which serves as an electron spin polarization source, is mixed into a target sample, and electron spin resonance is excited by high-frequency microwaves (100 to 300 GHz) or submillimeter waves (300 to 700 GHz), whereby it is possible to enhance NMR signals.

For example, NPL 1 discloses a technique for chemically bonding an organic radical polarizing agent to a ligand molecule that specifically binds to a target molecule, thereby selectively enhancing the NMR signal derived from the target molecule (protein) in in vitro measurements in a contaminated system that mimics the intracellular environment. Further, PTL 1 discloses a technique for analyzing the structure of a protein using a method of observing nitrogen-vacancy centers in nanodiamonds (NDs) bound to the protein using microscopic spectroscopy (ODMR method).

### Citation List

#### Patent Literature

PTL 1: WO2014/058012

#### Non-patent Literature

NPL 1: T. Viennet, A. Viegas, A. Kuepper, S. Arens, V. Gelev, O. Petrov, T.N. Grossmann, H. Heise, and M. Etzkorn, Angew. Chem. Int. Ed. 2016, 55, 10746-10750.

### Summary of Invention

#### Technical Problem

However, the organic radicals used in the method disclosed in NPL 1 and in many commercially available polarizing agents decompose in physiological reducing environments, making it difficult to achieve the desired NMR signal enhancement within cells. In addition, with the protein structure analysis method using the ODMR method disclosed in PTL 1, it is impossible to widely and generally increase the sensitivity of protein NMR spectra to perform structure analysis. No versatile and highly sensitive analysis method for intracellular proteins using NDs has been reported to date.

The present invention was made in view of the above circumstances, and an object thereof is to provide a polarizing agent that can selectively enhance NMR signals by the DNP-NMR method even in a reducing environment, and a method for selectively enhancing NMR signals.

#### Solution to Problem

The present inventors conducted extensive research to achieve the above object, and consequently found that the object can be achieved by using a nanodiamond whose surface is modified with a functional group capable of covalently binding to proteins, other drugs, or other organic molecules. The present invention has thus been completed.

Specifically, the present invention includes, for example, the subject matter described in the following items.

Item 1

A DNP-NMR polarizing agent that is a polarizing agent for use in a DNP-NMR method,

the DNP-NMR polarizing agent comprising nanodiamond particles, and

the nanodiamond particles having a surface modified with a functional group capable of covalently binding to or interacting with proteins.

Item 1'

A DNP-NMR polarizing agent that is a polarizing agent for use in a DNP-NMR method,

the DNP-NMR polarizing agent comprising nanodiamond particles, and
the nanodiamond particles having a surface modified with a functional group capable of covalently binding to proteins, drugs, or organic molecules, or with a functional group capable of interacting with proteins, other drugs, or other organic molecules.

Item 2

The DNP-NMR polarizing agent according to Item 1, wherein the functional group capable of covalently binding to drugs or organic molecules is at least one group selected from the group consisting of -C=C-, -COOH, $-NH_2$, -S-S-Py wherein Py represents a pyridine ring, and a group derived from biotin.

Item 2'

The DNP-NMR polarizing agent according to Item 1, wherein the functional group capable of interacting with proteins, drugs, or organic molecules is at least one group selected from the group consisting of -C=C-, -COOH, $-NH_2$, -S-S-Py wherein Py represents a pyridine ring, and a group derived from biotin.

Item 3

The DNP-NMR polarizing agent according to Item 1 or 2, wherein the nanodiamond particles are further modified with a charged group.

Item 3'

The DNP-NMR polarizing agent according to Item 1' or 2', wherein the nanodiamond particles are further modified with a charged group.

Item 4

The DNP-NMR polarizing agent according to any one of Items 1 to 3, wherein the functional group and a protein are bonded together.

Item 4'

The DNP-NMR polarizing agent according to any one of Items 1' to 3', wherein the functional group and a protein are bonded together.

Item 5

A method for producing the DNP-NMR polarizing agent according to any one of Items 1 to 3, comprising modifying nanodiamond particles modified with a reactive group with a compound having a functional group capable of covalently binding to proteins, drugs, or organic molecules, or with a compound having a functional group capable of interacting with proteins, drugs, or organic molecules.

Item 5'

A method for producing the DNP-NMR polarizing agent according to any one of Items 1' to 3', comprising modifying nanodiamond particles modified with a reactive group with a compound having a functional group capable of covalently binding to proteins, drugs, or organic molecules, or with a compound having a functional group capable of interacting with proteins, drugs, or organic molecules.

Item 6

A method for using the DNP-NMR polarizing agent according to any one of Items 1 to 3, comprising binding a target protein to a functional group capable of covalently binding to proteins, thereby enhancing an NMR signal.

Item 6'

A method for using the DNP-NMR polarizing agent according to any one of Items 1' to 3', comprising binding a target protein, another drug, or another organic molecule to a functional group capable of covalently binding to proteins, other drugs, or other organic molecules, thereby enhancing an NMR signal.

Item 6-1

A method for using the DNP-NMR polarizing agent according to any one of Items 1 to 3, comprising binding a target protein, another drug, or another organic molecule to a functional group capable of interacting with proteins, other drugs, or other organic molecules, thereby enhancing an NMR signal.

Advantageous Effects of Invention

The polarizing agent of the present invention can selectively enhance NMR signals by the DNP-NMR method even in a reducing environment.

Brief Description of Drawings

Fig. 1 shows specific examples of the DNP-NMR polarizing agent of the present invention.
Fig. 2 is a reaction scheme for obtaining the polarizing agent of Example 1.
Fig. 3 is a reaction scheme for obtaining the polarizing agent of Example 2.
Fig. 4 is a scheme that explains the summary of a method for confirming protein binding efficiency.
Fig. 5 shows the results of confirming whether the $^{13}$C-NMR signals of physiological small molecules of glycerol can be enhanced by using polarizing agent 1P as a DNP polarizing agent.
Fig. 6 (a) shows the $^{13}$C DNP-NMR spectra of protein (GB1) enhanced by polarizing agent 1P, and Fig. 6 (b) shows the $^{13}$C DNP-NMR spectra of protein (GB1) enhanced by polarizing agent 2P.
Fig. 7 shows the results of measuring the $^{13}$C DNP-NMR spectra (temperature: 30 K) of glycerol using a polarizing agent in the presence of a reducing agent.

Description of Embodiments

Below, embodiments of the present invention are described in detail. The terms "comprise," "contain," and "include" in the present specification include the concepts of "comprise," "contain," "include," "consist essentially of," and "consist of."

1. DNP-NMR Polarizing Agent

The polarizing agent of the present invention is a polarizing agent for use in the DNP-NMR method, the polarizing agent comprising nanodiamond particles, and the nanodiamond particles having a surface modified with a functional group capable of covalently binding to proteins, drugs, or organic molecules. Alternatively, the polarizing agent of the present invention is a polarizing agent for use in the DNP-NMR method, the polarizing agent comprising nanodiamond particles, and the nanodiamond particles having a surface modified with a functional group capable of interacting with proteins, drugs, or organic molecules. The polarizing agent of the present invention can selectively enhance NMR signals by the DNP-NMR method even in a reducing environment.

The polarizing agent contains nanodiamond particles whose surface is modified with a functional group capable of covalently binding to proteins, drugs, or organic molecules, or with a functional group capable of interacting with proteins, drugs, or organic molecules. Hereinafter, the "nanodiamond particles whose surface is modified with a functional group capable of covalently binding to proteins, drugs, or organic molecules, or with a functional group capable of interacting with proteins, drugs, or organic molecules" are referred to as "NDs." Further, the "functional group capable of covalently binding to proteins, drugs, or organic molecules" and the "functional group capable of interacting with

proteins, drugs, or organic molecules" are each referred to as "functional group F." NDs are preferably nanodiamond particles whose surface is modified with a functional group capable of covalently binding to or interacting with proteins.

ND is a particle with a structure having a nanodiamond as the core. In other words, ND has a structure in which a nanodiamond as the core is modified with functional group F. Hereinafter, the nanodiamond as the core is referred to as "the nanodiamond core."

The nanodiamond core has an average particle size of, for example, 100 nm or less, preferably 40 nm or less, and more preferably 10 nm or less. Further, the nanodiamond core has an average particle size of, for example, 1 nm or more. The "average particle size" as mentioned herein refers to a value measured by a dynamic light scattering method.

Functional group F chemically binds directly or indirectly to the nanodiamond core. More specifically, functional group F is present on the nanodiamond core surface directly or indirectly via a covalent bond.

The type of functional group F is not particularly limited, and any of a wide range of known functional groups can be used as long as it is a functional group capable of covalently binding to or interacting with proteins, drugs, or organic molecules. The "functional group capable of covalently binding to proteins" refers to a functional group that can form covalent bonds by reacting with functional groups of amino acids that constitute proteins. Examples of the functional groups of amino acids that constitute proteins include an amino group at the N-terminus, and a carboxy group, an amino group, a thiol group, or the like at the side chain. The "functional group capable of interacting with proteins" is, for example, a functional group derived from biotin that can interact with proteins, such as avidin. Functional group F is preferably a functional group capable of covalently binding to proteins or a functional group capable of interacting with proteins, and more preferably a functional group capable of covalently binding to proteins.

Of these, functional group F is preferably a group containing at least one member selected from the group consisting of -C=C-, -COOH, $-NH_2$, -S-S-Py wherein Py represents a pyridine ring, and a group derived from biotin. In this case, the target polarizing agent can be produced by a simple method, and the resulting polarizing agent can efficiently bond to proteins and has an excellent NMR signal enhancement effect.

When functional group F is -C=C-, specific examples include a maleimide group (a group formed by removing the hydrogen atom bonded to the N atom of maleimide). Therefore, when functional group F is -C=C-, -C=C- may be a group present in the ring structure. When functional group F is -S-S-Py, the -S-S-bond may be linked to any of positions 2, 3, and 4 of the pyridine ring, and examples include a group with an -S-S-S bond formed at position 2. When functional group F is a group derived from biotin, examples include a group formed by removing OH from the terminal carboxy group of biotin.

When functional group F indirectly binds to the nanodiamond core surface, a linker is present between functional group F and the nanodiamond core. The linker is, for example, a linear divalent group. Examples of the linear divalent group include a linear alkylene group. The length of the linker is not particularly limited, and can be appropriately set depending on the magnetic field, temperature, and sample type. The length of the linker is, for example, 1 to 50 nm. In this case, the target polarizing agent can be produced by a simple method, and the resulting polarizing agent has an excellent NMR signal enhancement effect. The length of the linker is preferably 3 to 10 nm, and more preferably about 5 nm. One end of the linker directly binds to the carbon atom in the nanodiamond.

NDs may be further modified with a spacer in addition to functional group F. The spacer as mentioned herein is a group that binds to the nanodiamond core directly or indirectly, and that is arranged between adjacent functional groups F. Due to the presence of such a spacer, functional group F is less likely to be dense on the ND surface, and an appropriate distance is maintained between adjacent functional groups F.

Therefore, NDs having a spacer can prevent excessive accumulation of the protein on the ND surface, thus can efficiently bind to the protein, and also have an excellent NMR signal enhancement effect.

The type of spacer is not particularly limited, and examples include a group derived from a linear or branched molecule. The linear or branched molecule preferably has a charge at the end (opposite side of the nanodiamond core).

That is, NDs (nanodiamond particles) are preferably further modified with a charged group. In this case, due to charge repulsion between NDs, dispersibility in solvents is likely to be improved. The charged group may be a group other than functional group F, or the same type of group as functional group F. The charge may be positive or negative, and a negative charge is preferred because dispersibility is more likely to be improved. Examples of negatively charged groups include -COOH, $-SO_3H$, $-PO_3$, and the like, and examples of positively charged groups include $-NH_2$ and the like.

Specific examples of the spacer include a group derived from a molecule represented by the following formula (1):

$$NH_2\text{-}X \qquad (1)$$

In formula (1), examples of X include monovalent groups represented by the following formulas (1-1), (1-2), and (1-3):

$$-(CH_2)_a\text{-}SO_3H \qquad (1\text{-}1)$$

$$-(CH_2)_a-(OCH_2CH_2)_b-OH \quad (1\text{-}2)$$

$$-(CH_2)_a-NH_2 \quad (1\text{-}3)$$

a is, for example, 1 to 10, and preferably 1 to 5. b is, for example, 1 to 10, and preferably 1 to 5.

When the spacer is the group derived from the molecule represented by formula (1), the end of the amino group side in formula (1) is placed on the nanodiamond core side. Specifically, the nitrogen atom from which the hydrogen atoms of the amino group are removed is covalently bonded to the nanodiamond surface. However, when one end is a hydroxyl group as in formula (1-2), the oxygen atom from which the hydrogen atom of the hydroxyl group is removed can be covalently bonded to the nanodiamond surface.

NDs may be modified with a single type of spacer or two or more types of spacers.

When functional group F is covalently bonded to the nanodiamond surface through the linker, it is preferable that the distance from the end of the spacer to the nanodiamond core surface is shorter than the distance from functional group F to the nanodiamond core surface in terms of easy protein binding.

In NDs, the content of the spacer is not particularly limited. For example, the content of the spacer can be 0.1 to 100 mol, preferably 0.5 to 80 mol, and preferably 1 to 50 mol, per mol of functional group F.

Formulas (2-1), (2-2), (2-3), (2-4), and (2-5) shown in Fig. 1 schematically show specific examples of NDs.

In formula (2-3), m is 1 to 10, and preferably 1 to 5. In formula (2-5), n is 1 to 10, and preferably 1 to 5. The wavy lines in formulas (2-1), (2-2), (2-3), (2-4), and (2-5) each mean a direct covalent bond to the nanodiamond core surface, and in this case, also mean a direct covalent bond to the functional group on the nanodiamond core surface. The functional group on the nanodiamond core surface is, for example, reactive group R described later.

As the DNP-NMR polarizing agent of the present invention, a compound produced by the reaction of a nanodiamond having reactive group R on its surface and compound C described later can be preferably used.

The DNP-NMR polarizing agent of the present invention may have a structure in which functional group F binds to a protein, a drug, or an organic molecule (i.e., in the DNP-NMR polarizing agent of the present invention, a protein, a drug, or an organic molecule may be bonded to functional group F contained in NDs). Therefore, the DNP-NMR polarizing agent of the present invention also includes polarizing agents in which a protein, a drug, or an organic molecule is bonded to functional group F contained in NDs, in addition to NDs.

The type of protein is not particularly limited, and any of a wide range of known proteins can be applied as long as it can chemically react with functional group F to form a covalent bond, or can interact with functional group F. Drugs or organic molecules are other than proteins, and examples include small molecules such as biotin, which is commonly used in intracellular protein detection tests, drug molecules such as clenbuterol, which is an agonist of the cell surface receptor GPCR, enzyme substrate molecules such as acetazolamide, which is a ligand for decarboxylase, and sugar molecules such as β-galactose, which is involved in immune responses.

For example, when functional group F is -C=C-, for example, the thiol group (e.g., cysteine) present in the protein is reacted with functional group F (e.g., thiol-ene reaction), whereby the protein covalently binds to the ND surface. For example, when functional group F is -COOH, the amino group present in the protein is reacted with functional group F, whereby the protein covalently binds to the ND surface. For example, when functional group F is $-NH_2$, the carboxy group present in the protein is reacted with functional group F, whereby the protein covalently binds to the ND surface. For example, when functional group F is -S-S-Py, the thiol group (e.g., cysteine) present in the protein is reacted with functional group F, whereby the protein covalently binds to the ND surface. For example, when functional group F is a group derived from biotin, the avidin moiety present in the protein interacts with functional group F, whereby the protein non-covalently binds to the ND surface.

The DNP-NMR polarizing agent of the present invention may consist of NDs, or may consist of NDs binding to a protein, another drug, or another organic molecule, or may comprise other polarizing agents as long as the effects of the present invention are not impaired. It is preferable for the DNP-NMR polarizing agent of the present invention to contain NDs or protein-binding NDs in an amount of 50 mass% or more, more preferably 80 mass% or more, even more preferably 90 mass% or more, and still even more preferably 99 mass% or more.

Due to the above configuration, the DNP-NMR polarizing agent of the present invention has excellent reduction resistance. That is, the use of the DNP-NMR polarizing agent of the present invention enables in-situ tracking of protein structures and chemical reactions even in a strong reducing environment, and enables functional measurement of bioactive substances even in in vitro experiments under conditions more similar to the in vivo environment.

Conventionally, TEMPOL radical widely used as a polarizing agent has been rapidly decomposed in the presence of reducing biomolecules, such as ascorbic acid and glutathione (e.g., within cells), whereas the electron spins of the DNP-NMR polarizing agent of the present invention are protected by a bulky, kinetic-poor carbon skeleton, which provides excellent reduction resistance.

Since the DNP-NMR polarizing agent of the present invention is used in DNP-NMR measurements, it is possible to directly obtain a wide variety of atomically resolved information on the chemical and steric structures of proteins, such

as chemical shift values and inter-nuclear distance information, with high sensitivity (e.g., multi-dimensional decomposition correlation measurement can be used). In this respect, the DNP-NMR method using the DNP-NMR polarizing agent of the present invention has great advantages over the conventional ODMR method (see, for example, PTL 1 mentioned above).

By introducing the DNP-NMR polarizing agent containing a protein binding to NDs into a living cell, for example, by electroporation, it is possible to directly obtain protein structure information from chemical shift values, inter-nuclear distance information, and tensor relative orientation information in a strongly reducing environment of the cells, with atomic resolution. That is, it is expected that the use of multi-dimensional decomposition correlation measurement will make it possible to collect a wide variety of information at once and to clarify the distribution of protein function, dynamics, and dynamic equilibrium structure in the cells (at the site of function). By performing the above multi-dimensional decomposition correlation measurement before and after the introduction of ligand molecules related to the cell signaling of interest into cells, it is possible to detect and quantify the structure response of the relevant protein in the cells.

It is also possible to modify the ND surface with a drug-biomaterial of a drug discovery seed or a signal peptide, which is an intracellular address, deliver it to a specific binding partner protein at a specific site in the foreign cell, and obtain a highly sensitive NMR spectra specifically to the intracellular site. In addition, the fluorescence emission properties inherent in NDs can be used to obtain information on the localization of target proteins in cells by fluorescence microscopy.

2. Method for Producing DNP-NMR Polarizing Agent

The method for producing the DNP-NMR polarizing agent of the present invention is not particularly limited. For example, the NDs described above can be produced by a production method comprising the following step 1.

Step 1: modifying nanodiamond particles modified with a reactive group with a compound having a functional group capable of covalently binding to proteins, drugs, or organic molecules, or with a compound having a functional group capable of interacting with proteins, drugs, or organic molecules.

Hereinafter, the reactive group in step 1 is referred to as "reactive group R," and the compound having a functional group capable of covalently binding to proteins, or the compound having a functional group capable of interacting with proteins, drugs, or organic molecules, is referred to as "compound C."

In the nanodiamond particles modified with reactive group R used in step 1, the type of reactive group R is not particularly limited, and examples include a carboxy group, an amino group, a hydroxyl group, and the like. The nanodiamond particles modified with such reactive group R can be obtained by a known method, or can be obtained from a commercial product etc.

For example, when reactive group R is a carboxy group, a commercially available carboxy group-modified nanodiamond can be used as such nanodiamond particles. The carboxy group-modified nanodiamond can be separately synthesized. For example, carboxy group-modified nanodiamond particles can be obtained by reacting a hydroxyl group-modified nanodiamond, an amino group-modified nanodiamond, or an aldehyde group-modified nanodiamond, with an acid anhydride (e.g., succinic anhydride).

The nanodiamond particles modified with reactive group R have an average particle size of, for example, 100 nm or less, preferably 40 nm or less, and more preferably 30 nm or less. Further, the nanodiamond core has an average particle size of, for example, 1 nm or more. The "average particle size" as mentioned herein refers to a value measured by a dynamic light scattering method.

In compound C, the functional group is synonymous with functional group F described above. Therefore, compound C also has functional group F. Functional group F in compound C is preferably at least one group selected from the group consisting of -C=C-, -COOH, $-NH_2$, -S-S-Py wherein Py represents a pyridine ring, and a group derived from biotin.

When functional group F in compound C is -C=C-, specific examples include a maleimide group (a group formed by removing the hydrogen atom bonded to the N atom of maleic acid). When functional group F in compound C is -S-S-Py, as a specific example, the -S-S- bond may be linked to any of positions 2, 3, and 4 of the pyridine ring, and examples include a group with an -S-S-S bond formed at position 2. When functional group F in compound C is a group derived from biotin, examples include a group formed by removing OH from the terminal carboxy group of biotin.

Compound C has functional group F in the molecule, and further has functional group A that can react with reactive group R described above. Examples of such functional group A include an amino group, a carboxy group, $-NH_3^+$, and the like.

Specific examples of compound C include the following formulas (C-1), (C-2), (C-3), and (C-4).

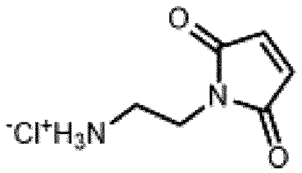

(C－1)

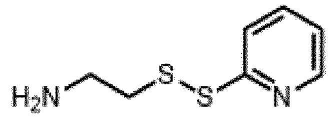

(C－2)

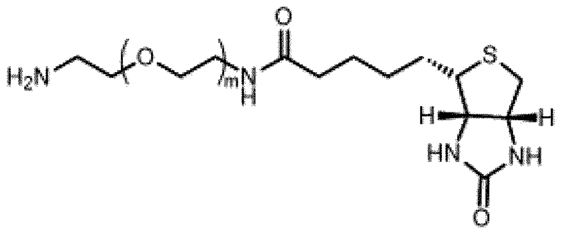

(C－3)

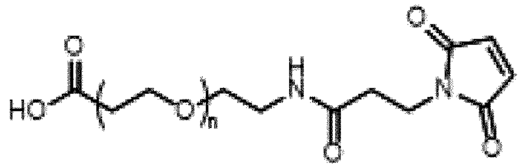

(C－4)

In step 1, the nanodiamond particles modified with reactive group R are modified with compound C. As a result, NDs with a nanodiamond core modified with functional group F are obtained.

In step 1, the method for modifying the nanodiamond particles with compound C is not particularly limited, and, for example, any of a wide range of known methods can be used. For example, when a carboxy group-modified nanodiamond is used, a known condensation reaction can be used. Specifically, for example, the carboxy group of the carboxy group-modified nanodiamond is reacted with a condensing agent, and then reacted with an active reagent to produce a precursor, which is then reacted with compound C. In compound C in this case, functional group A is preferably an amino group or $NH_3^+$.

As the condensing agent, any of a wide range of known condensing agents for use in condensation reactions can be used, and examples include N,N'-dicyclohexylcarbodiimide (DCC) and 1-ethyl-3-(3-dimethylaminopropyl)carbodiimide hydrochloride (EDC). Further, examples of the active reagent include N-hydroxysuccinimide (NHS), N-hydroxysulfosuccinimide sodium (Sulfo-NHS), and the like.

Further, when an amino group-modified nanodiamond is used, a known condensation reaction can be used. For example, a precursor produced by reacting an amino group-modified nanodiamond with an active reagent similar to the above is reacted with compound C. In compound C in this case, functional group A is preferably a carboxy group.

In step 1, a spacer molecule can also be used in combination with compound C. The combined use of such a spacer molecule can give NDs modified with functional group F and a spacer.

Examples of the spacer molecule include linear or branched molecules. Such a linear or branched spacer molecule preferably has a charge at the end. In this case, due to charge repulsion between NDs, dispersibility in solvents is likely to be improved. The charge may be positive or negative, and a negative charge is preferred because dispersibility is more likely to be improved. Therefore, examples of the end of the spacer molecule include ionized groups, such as $-COOH$, $-SO_3H$, $-NH_2$, and $-PO_3$.

Specific examples of the spacer molecule include the compound represented by formula (1) described above. When the spacer molecule is represented by formula (1), reactive group R on the nanodiamond particle surface is preferably a carboxy group. In this case, the amino group side terminal of the compound represented by formula (1) reacts with reactive group R on the nanodiamond particle surface to form an amide bond, and the surface is modified with the spacer.

In step 1, only one type of spacer molecule can be used, or two or more types of spacer molecules can be used.

In step 1, the amount of the spacer used is not particularly limited. For example, the amount of the spacer used can be 0.1 to 100 mol, preferably 0.5 to 80 mol, and preferably 1 to 50 mol, per mol of functional group F.

The condensation reaction and the modification of compound C in step 1 can be performed, for example, in a solvent. Water can be used as the solvent. In addition, an organic solvent or a mixed solvent of water and an organic solvent can also be used as long as the reaction is not inhibited. When the reaction is performed in a solvent, the concentration of raw materials etc. is not particularly limited, and can be the same as in a known reaction.

NDs can be obtained by step 1 described above. NDs can be reacted with a protein, thereby obtaining a DNP-NMR polarizing agent having a structure in which functional group F and the protein are bonded together. The reaction conditions between NDs and the protein are also not particularly limited. For example, a wide range of known reaction conditions for functional group F and proteins can be used.

3. Method for Using DNP-NMR Polarizing Agent

The DNP-NMR polarizing agent of the present invention can be used as a polarizing agent in DNP-NMR measurements.

For example, when the DNP-NMR polarizing agent of the present invention is NDs described above, an embodiment of the method for using NDs is, for example, a use method in which a target protein, another drug, or another organic molecule is bonded to a functional group capable of covalently binding to proteins, drugs, or organic molecules, thereby enhancing NMR signals. Alternatively, another embodiment of the method for using NDs is, for example, a use method in which a target protein, another drug, or another organic molecule is bonded to a functional group capable of interacting with proteins, other drugs, or other organic molecules, thereby enhancing NMR signals.

The method for binding a target protein, another drug, or another organic molecule to functional group F is not particularly limited. For example, a wide range of known reaction conditions for functional group F and proteins can be used. The binding efficiency of the protein to functional group F can be confirmed and quantified by a protein assay using the bicinchoninic acid method (BCA method).

When the DNP-NMR polarizing agent of the present invention is one in which a protein binds to functional group F in the NDs, such a polarizing agent has reduction resistance. Therefore, it can be introduced into living cells to directly analyze the structure of proteins. For example, the ND surface is modified with a drug-biomaterial of a drug discovery seed or a signal peptide, which is an intracellular address, and delivered to a specific binding partner protein present at a specific site in the foreign cell. This makes it possible to obtain a highly sensitive NMR spectra specifically to the intracellular site, and also obtain information on the localization of target proteins in cells.

Examples

Below, the present invention is described in more detail with reference to Examples. However, the present invention is not limited to the embodiments of the Examples.

Example 1

Polarizing agent 1 containing nanodiamond particles was prepared according to the reaction scheme shown in Fig. 2. First, monodispersed nanodiamond particles whose surface was modified with a carboxy group and having an average particle size of 5 nm (Sigma-Aldrich Monodispersed nanodiamond particles 5 nm avg. part. size (DLS), 10 mg/mL in $H_2O$, carboxylated) were prepared. 11.62 mg of 1-ethyl-3-(3-dimethylaminopropyl)carbodiimide hydrochloride (EDC) and 13.19 mg of N-hydroxysulfosuccinimide sodium (Sulfo-NHS) were added to 1.0 mL of a 10 mg/mL aqueous dispersion of the nanodiamond particles, followed by reaction by stirring for 30 minutes, thereby obtaining a reaction liquid. In this reaction, the nanodiamond particles were reacted with EDC to produce precursor a, and precursor a was reacted with Sulfo-NHS to produce precursor b (see Fig. 2).

6.77 mg of taurine as a spacer molecule and 1.05 mg of N-(2-aminoethyl)maleimide hydrochloride as compound C were added to the reaction liquid, followed by reaction by stirring overnight, thereby obtaining a reactant containing polarizing agent 1. The reactant was subjected to dialysis purification with ultrapure water using a dialysis membrane with a cutoff molecular weight of 8000 Da to efficiently redisperse aggregates formed after the reaction. The introduction of sulfone groups into polarizing agent 1 was confirmed by infrared spectroscopy. Further, the introduction of maleimide groups into polarizing agent 1 was presumed from the fact that in addition to the peaks derived from sulfone groups ($-SO_3H$) of taurine at 1040 $cm^{-1}$ and 1220 $cm^{-1}$, the peak of carbonyl groups (C=O) at 1630 $cm^{-1}$ increased in the infrared absorption spectrum. As described later, maleimide groups were considered to be introduced from the fact that the protein bound to polarizing agent 1. In Fig. 2, m:n was set to 11:100.

Example 2

Polarizing agent 2 containing nanodiamond particles was prepared according to the reaction scheme shown in Fig. 3. First, monodispersed nanodiamond particles whose surface was modified with a carboxy group and having an average particle size of 5 nm (Sigma-Aldrich Monodispersed nanodiamond particles 5 nm avg. part. size (DLS), 10 mg/mL in $H_2O$, carboxylated) were prepared. 1.0 mL of a 10 mg/mL aqueous dispersion of the nanodiamond particles was prepared, and 19.71 mg of 1-ethyl-3-(3-dimethylaminopropyl)carbodiimide hydrochloride (EDC) and 21.75 mg of

N-hydroxysulfosuccinimide sodium (Sulfo-NHS) were added to the aqueous dispersion, followed by reaction by stirring for 30 minutes, thereby obtaining a reaction liquid. The reaction liquid was added dropwise to 1.0 mL of ethylene diamine over 5 minutes to obtain an amino group-modified ND dispersion (see Fig. 3). The reactant was subjected to dialysis purification with ultrapure water using a dialysis membrane with a cutoff molecular weight of 8000 Da to efficiently redisperse aggregates formed after the reaction.

Then, 2.49 mg of raw material a (see Fig. 3) as compound C, 1.50 mg of EDC, 1.88 mg of Sulfo-NHS, and 3.0 mL of purified water were added, followed by reaction by stirring for 30 minutes, and the reaction liquid was added to the amino group-modified ND dispersion, thereby obtaining a reactant containing polarizing agent 2. The reactant was subjected to dialysis purification with ultrapure water using a dialysis membrane with a cutoff molecular weight of 8000 Da to efficiently redisperse aggregates formed after the reaction. In Fig. 3, m:n was set to 100:6.

Test Example 1: Binding between Polarizing Agent 1 and Target Protein

Polarizing agent 1 and an aqueous solution of a target protein (GB1T53C) obtained by replacing the 53rd threonine residue of the B1 domain of protein G expressed from *E. coli* with cysteine were mixed and stirred overnight to obtain a reaction liquid. Then, in order to remove the unreacted protein, tris(2-carboxyethyl)phosphine hydrochloride (TCEP) was added so that the concentration of the reaction liquid was 5 mM, dialysis purification was performed using the 5 mM TCEP aqueous solution, and then dialysis purification was performed using ultrapure water to remove TCEP. As a result, polarizing agent 1P formed such that functional group F (-C=C- in the maleimide group) of polarizing agent 1 was reacted with the -SH group of the target protein (see Fig. 2) was obtained.

Test Example 2: Binding between Polarizing Agent 2 and Target Protein

Polarizing agent 2P formed such that functional group F (-C=C- in the maleimide group) of polarizing agent 2 was reacted with the -SH group of the target protein (see Fig. 3) was obtained in the same manner as in Test Example 1, except that polarizing agent 1 was changed to polarizing agent 2.

Confirmation Test of Protein Binding Efficiency

According to the scheme shown in Fig. 4, the binding efficiency of polarizing agents 1 and 2 and the target protein was confirmed and quantified by a protein assay using the bicinchoninic acid method (BCA method). Although BCA does not react with $Cu^{2+}$, it forms a complex with $Cu^{+}$ generated by the biuret reaction and shows absorption at 562 nm. Thus, the absorbance at 562 nm is compared with that of a standard sample, such as albumin, to determine the protein content. However, in this test, the ratio of absorbance was taken before and after dialysis purification to evaluate the reaction efficiency without using a standard sample. The BCA test reagent used was a commercially available kit. The reaction efficiency was calculated by the following formula:

$$\text{Reaction efficiency (\%)} = [(Z-B)/\{((Y-B)/X)\}]\times 100$$

In the formula, X is the molar ratio of the protein binding site to the maleimide group, Y is the absorbance at 562 nm of the polarizing agent 1 solution (or the polarizing agent 2 solution) containing the unreacted protein immediately after the reaction, Z is the absorbance at 562 nm when the polarizing agent 1 solution (or the polarizing agent 2 solution) was dialyzed with the 5 mM TCEP aqueous solution and then dialyzed with ultrapure water, and B is the absorbance at 562 nm of the BCA reagent in ultrapure water.

The reaction efficiency calculated from the above formula was about 80% for polarizing agent 1P of Test Example 1 and about 63% for polarizing agent 2P of Test Example 2.

NMR Signal Enhancement Test of Nanodiamond Particles

Fig. 5 shows the results of confirming whether the $^{13}C$-NMR signals of physiological small molecules of glycerol can be enhanced by using the commercially available monodispersed nanodiamond particles mentioned above as a DNP polarizing agent. The DNP-NMR method was tested with an external magnetic field of 16.4 T, a temperature of 40 K, and a submillimeter wave frequency of 460 GHz. In Fig. 5, "MW=off" means under submillimeter-wave non-irradiation conditions, and "MW=on" means under submillimeter wave irradiation conditions. It was confirmed from Fig. 5 that the DNP polarizing agent enhanced the NMR signals of glycerol.

Fig. 6 (a) shows the $^{13}C$ DNP-NMR spectra of protein GB1 enhanced by polarizing agent 1P, and Fig. 6 (b) shows the $^{13}C$ DNP-NMR spectra of protein GB1 enhanced by polarizing agent 2P. In these figures, the solid line is the

NMR spectrum in the absence of microwave irradiation, the broken line is the NMR spectrum when positive DNP occurs under 459.8 GHz microwave irradiation, and the dotted line is the NMR spectrum when negative DNP occurs under 460.0 GHz microwave irradiation.

The obtained DNP-NMR spectra are derived from the proteins bound to polarizing agents 1 and 2, and the intensity changes upward and downward under microwave irradiation due to the effects of DNP. For polarizing agent 1P, a +69% increase and a -46% decrease in signal at 180 ppm were observed, corresponding to positive and negative DNP effects, respectively. For polarizing agent 2P, a +87% increase and a -144% decrease in signal at 180 ppm were observed. These results suggest that the length of the linker between the protein and NDs contributes to DNP efficiency (the length of the linkers in polarizing agents 1P and 2P is about 1 nm and about 3 nm, respectively).

Since "sensitivity enhancement" is usually desired, many measurements will be made under 459.8 GHz microwave irradiation causing positive DNP. The required microwave frequency is determined by the external magnetic field strength of NMR. In the test shown in Fig. 6, microwaves around 460 GHz were used for a magnetic field of 16.4 T. High-field NMR, which is commonly used for high-resolution measurements, requires 250 to 790 GHz microwaves or submillimeter waves for magnetic fields of 9 T to 28 T, based on a similar proportionality relation.

Confirmation of Reduction Resistance

Fig. 7 shows the results of measuring the $^{13}C$ DNP-NMR spectra (temperature: 30 K) of glycerol using the commercially available monodispersed nanodiamond particles mentioned above as a polarizing agent in the presence of 5 mM ascorbic acid as a reducing agent. In the figure, (1) shows the NMR signal of glycerol in the absence of microwave irradiation and in the presence of ascorbic acid, (2) shows the NMR signal of glycerol in the absence of microwave irradiation and in the absence of ascorbic acid, (3) shows the NMR signal of glycerol under 460 GHz microwave irradiation and in the presence of ascorbic acid, and (4) shows the NMR signal of glycerol under 460 GHz microwave irradiation and in the absence of ascorbic acid.

Fig. 7 shows that the NMR signal (1) matches well with the NMR signal (2), indicating that ascorbic acid does not induce structural changes or chemical reactions of glycerol. The NMR signals (3) and (4) were both enhanced downward showing negative DNP. The fact that the downward signal intensity is equal in the presence and absence of ascorbic acid indicates that the function of the polarizing agent ND as a polarizing agent is not impaired even in a reducing environment.

Industrial Applicability

The DNP-NMR polarizing agent of the present invention, which is a nanodiamond-based polarizing agent with high reduction resistance, enables in-situ tracking of protein structures and chemical reactions in a contaminated system, in a strong reducing environment, and in cells, which have been unobservable so far, and enables functional measurement of bioactive substances even in in vitro experiments under conditions more similar to the in vivo environment. Therefore, the DNP-NMR polarizing agent of the present invention can contribute to target discovery and drug discovery research.

## Claims

1. A DNP-NMR polarizing agent that is a polarizing agent for use in a DNP-NMR method,

   the DNP-NMR polarizing agent comprising nanodiamond particles, and
   the nanodiamond particles having a surface modified with a functional group capable of covalently binding to proteins, drugs, or organic molecules, or with a functional group capable of interacting with proteins, drugs, or organic molecules.

2. The DNP-NMR polarizing agent according to claim 1, wherein the functional group capable of covalently binding to drugs or organic molecules, and the functional group capable of interacting with proteins, drugs, or organic molecules are each at least one group selected from the group consisting of -C=C-, - COOH, $-NH_2$, -S-S-Py wherein Py represents a pyridine ring, and a group derived from biotin.

3. The DNP-NMR polarizing agent according to claim 1, wherein the nanodiamond particles are further modified with a charged group.

4. The DNP-NMR polarizing agent according to any one of claims 1 to 3, wherein the functional group and a protein are bonded together.

**5.** A method for producing the DNP-NMR polarizing agent according to any one of claims 1 to 3, comprising modifying nanodiamond particles modified with a reactive group with a compound having a functional group capable of covalently binding to proteins, drugs, or organic molecules, or with a compound having a functional group capable of interacting with proteins, drugs, or organic molecules.

**6.** A method for using the DNP-NMR polarizing agent according to any one of claims 1 to 3, comprising binding a target protein to a functional group capable of covalently binding to proteins, thereby enhancing an NMR signal.

Fig. 1

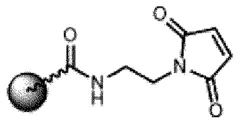

(2－1)

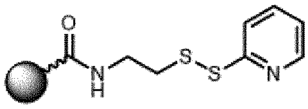

(2－2)

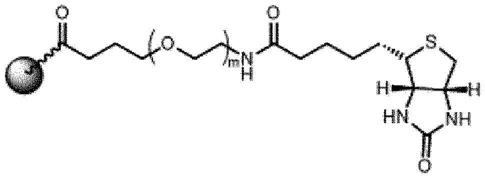

(2－3)

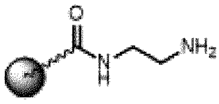

(2－4)

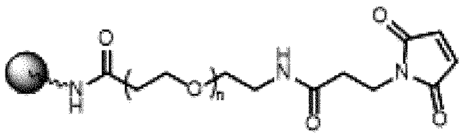

(2－5)

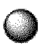 ≡ Nanodiamond core

Fig. 2

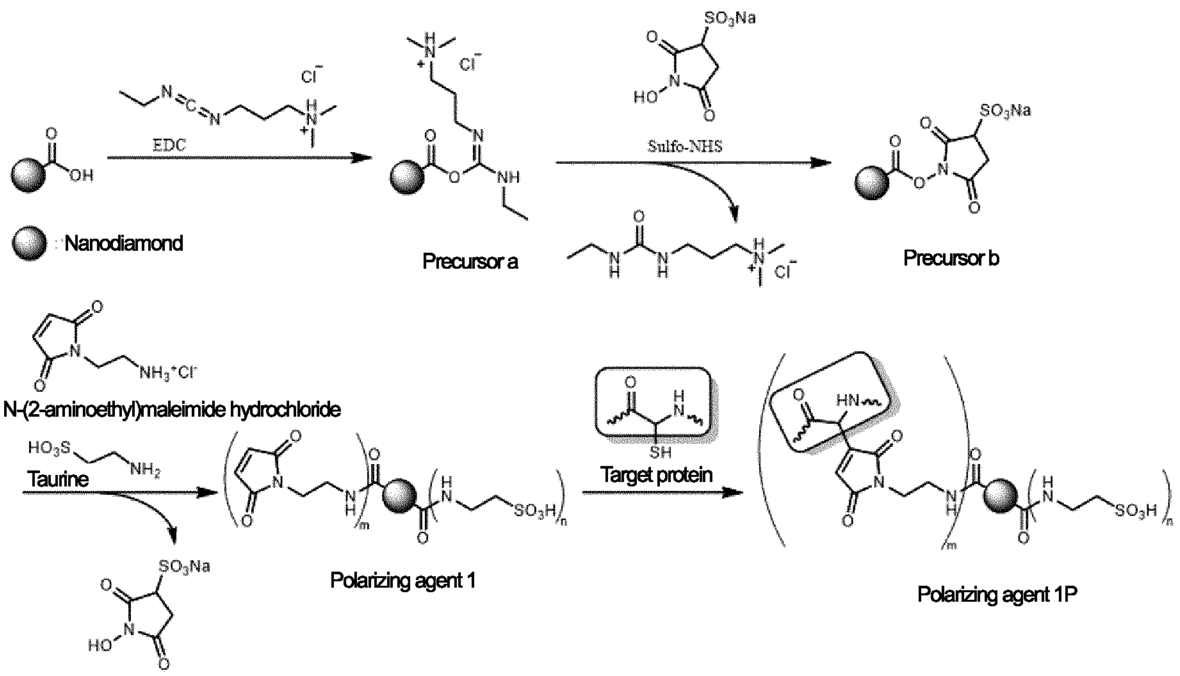
EDC
Sulfo-NHS
Nanodiamond
Precursor a
Precursor b
N-(2-aminoethyl)maleimide hydrochloride
Taurine
Target protein
Polarizing agent 1
Polarizing agent 1P

Fig. 3

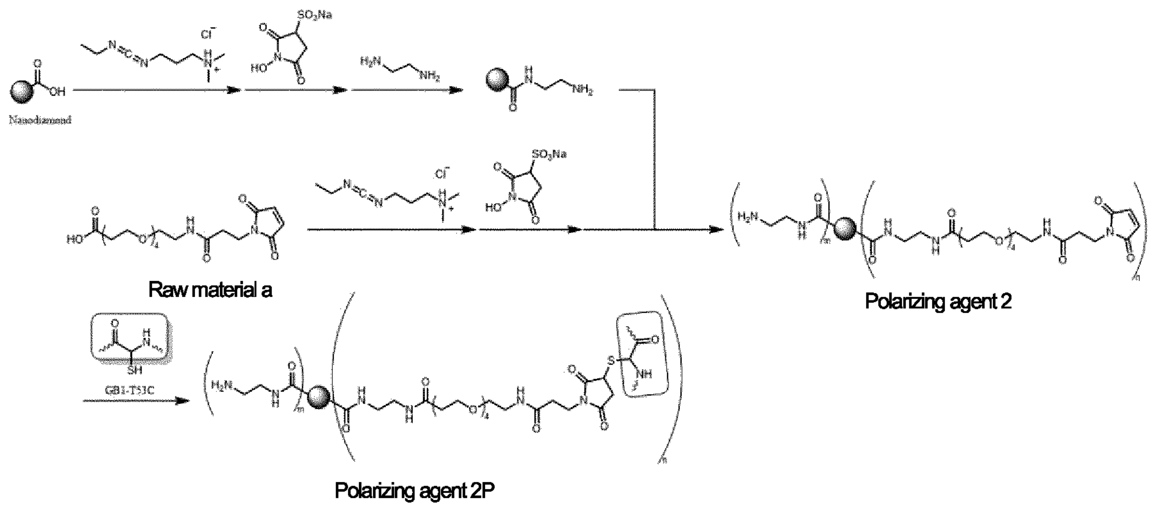
Nanodiamond
Raw material a
GB1-T53C
Polarizing agent 2
Polarizing agent 2P

Fig. 4

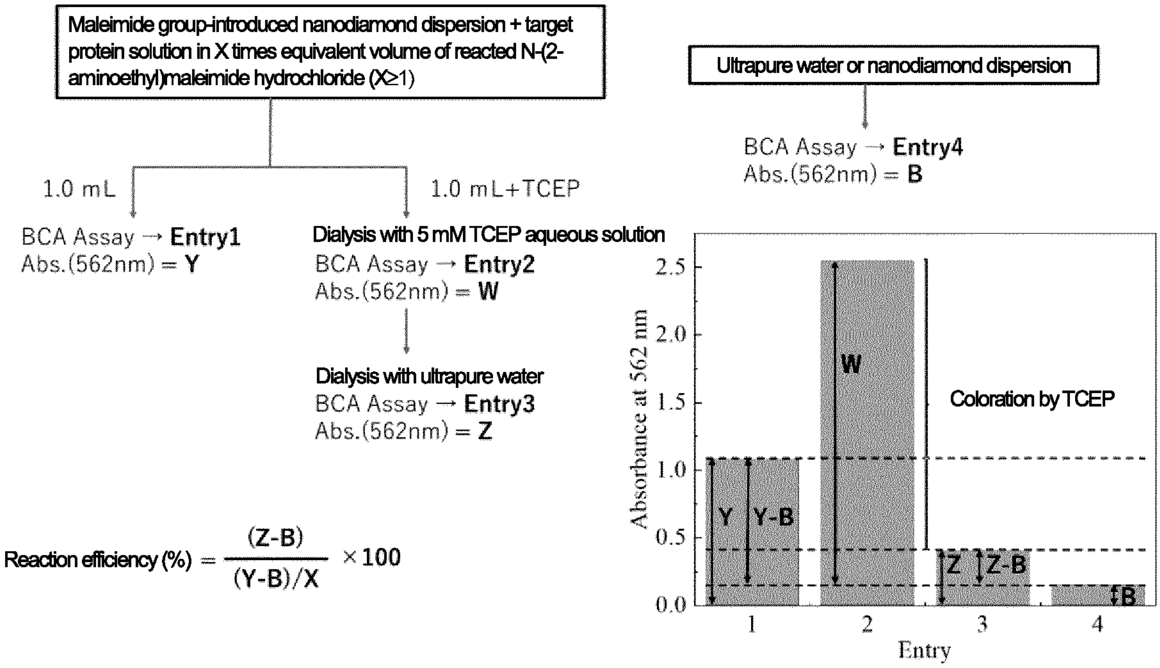
Maleimide group-introduced nanodiamond dispersion + target protein solution in X times equivalent volume of reacted N-(2-aminoethyl)maleimide hydrochloride (X≥1)
1.0 mL
BCA Assay → Entry1
Abs.(562nm) = Y
1.0 mL+TCEP
Dialysis with 5 mM TCEP aqueous solution
BCA Assay → Entry2
Abs.(562nm) = W
Dialysis with ultrapure water
BCA Assay → Entry3
Abs.(562nm) = Z
Ultrapure water or nanodiamond dispersion
BCA Assay → Entry4
Abs.(562nm) = B
Reaction efficiency (%) = (Z-B) / ((Y-B)/X) × 100
Absorbance at 562 nm
Coloration by TCEP
Y
Y-B
W
Z
Z-B
B
Entry
0.0
0.5
1.0
1.5
2.0
2.5
1
2
3
4

Fig. 5

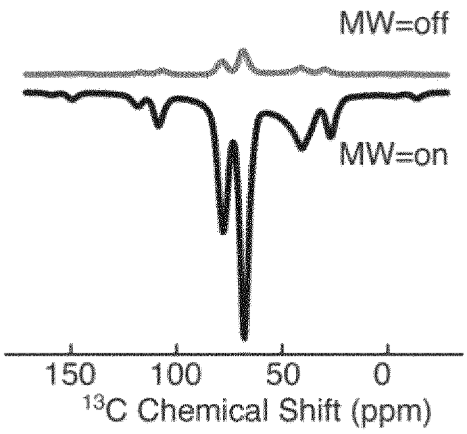
MW=off
MW=on
150
100
50
0
13C Chemical Shift (ppm)

Fig. 6

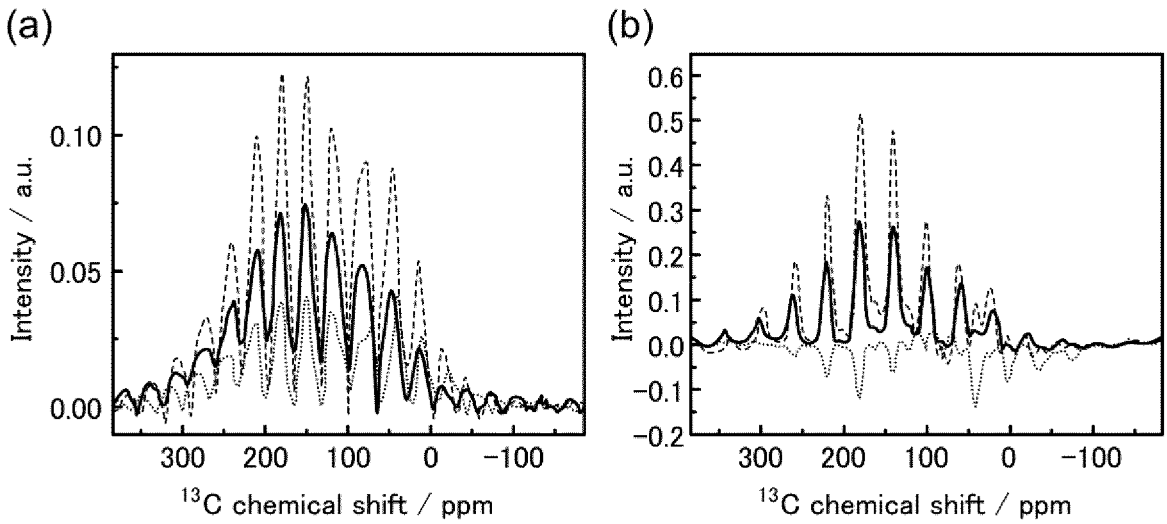
(a)
Intensity / a.u.
0.10
0.05
0.00
300
200
100
0
−100
$^{13}$C chemical shift / ppm
(b)
0.6
0.5
0.4
0.3
0.2
0.1
0.0
−0.1
−0.2
$^{13}$C chemical shift / ppm

Fig. 7

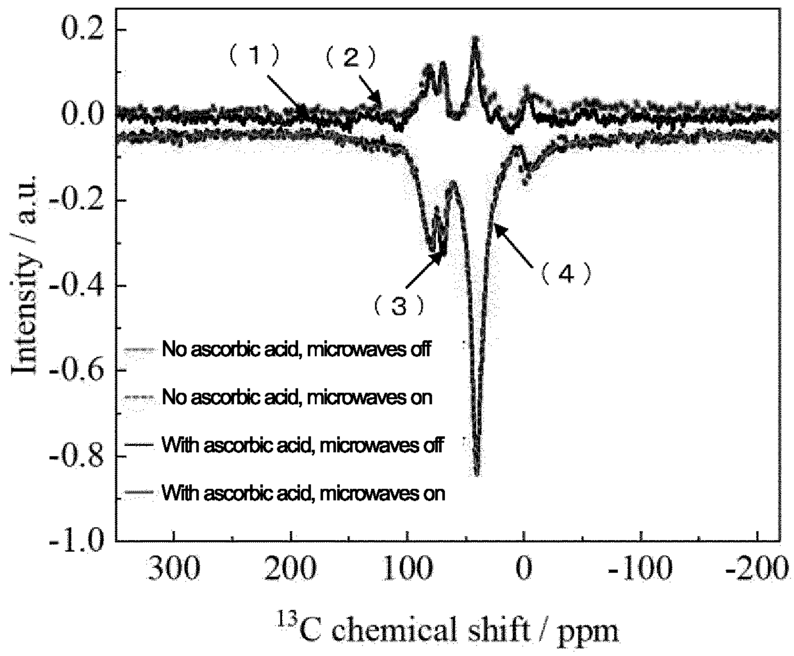
0.2
0.0
-0.2
-0.4
-0.6
-0.8
-1.0
Intensity / a.u.
(1)
(2)
(3)
(4)
No ascorbic acid, microwaves off
No ascorbic acid, microwaves on
With ascorbic acid, microwaves off
With ascorbic acid, microwaves on
300
200
100
0
-100
-200
$^{13}$C chemical shift / ppm

## INTERNATIONAL SEARCH REPORT

International application No. **PCT/JP2022/031633**

**A. CLASSIFICATION OF SUBJECT MATTER**

***G01N 24/08***(2006.01)i
FI: G01N24/08 510Z

According to International Patent Classification (IPC) or to both national classification and IPC

**B. FIELDS SEARCHED**

Minimum documentation searched (classification system followed by classification symbols)

G01N24/00-G01N24/14, G01R33/00-G01R33/64, A61B5/055

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII), Science Direct, Wiley Online Library, ACS PUBLICATIONS, nature.com, Oxford Journals, APS Journals, arXiv, KAKEN

**C. DOCUMENTS CONSIDERED TO BE RELEVANT**

| Category* | Citation of document, with indication, where appropriate, of the relevant passages | Relevant to claim No. |
|---|---|---|
| Y | On The Potential of Dynamic Nuclear Polarization Enhanced Diamonds in Solid-State and Dissolution 13C NMR Spectroscopy. ChemPhysChem. 15 July 2016, vol. 17, issue 17, pp. 2691-2701, doi:10.1002/cphc.21600301<br>3. Discussion and conclusions, fig. 1-7, supporting figures S1-S3 | 1-6 |
| Y | The properties and applications of nanodiamonds. Nature Nanotechnology. 18 December 2011, vol. 7, pp. 11-23, doi:10.1038/NNANO.2011.209<br>fig. 1-7 | 1-6 |
| A | Recent developments in MAS DNP-NMR of materials. Solid State Nuclear Magnetic Resonance. 27 May 2019, vol. 101, pp. 116-143, doi:10.1016/j.ssnmr.2019.05.009<br>3. Polarizing agents and sample preparation | 1-6 |
| A | Dendric polarizing agents for DNP SENS. Chemical Science. 22 August 2016, vol. 8, issue 1, pp. 416-422, doi:10.1039/c6sc03139k<br>fig. 1, 2, S1-S10 | 1-6 |
| A | US 2021/0179581 A1 (OHIO STATE INNOVATION FOUNDATION) 17 June 2021 (2021-06-17) | 1-6 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 September 2022** | **20 September 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/031633**

**C. DOCUMENTS CONSIDERED TO BE RELEVANT**

| Category* | Citation of document, with indication, where appropriate, of the relevant passages | Relevant to claim No. |
|---|---|---|
| A | JP 2018-514795 A (UNIVERSITAET ULM) 07 June 2018 (2018-06-07) | 1-6 |
| A | US 2018/0113185 A1 (THE GENERAL HOSPITAL CORPORATION) 26 April 2018 (2018-04-26) | 1-6 |
| T | Dynamic Nuclear Polarization Solid-State NMR Spectroscopy for Materials Research. Annual Review of Materials Research. 08 March 2022, 52:25-55, doi:10.1146/annurev-matsci-081720-085634<br>fig. 1-7 | 1-6 |

Form PCT/ISA/210 (second sheet) (January 2015)

**INTERNATIONAL SEARCH REPORT**
**Information on patent family members**

International application No.
**PCT/JP2022/031633**

| Patent document cited in search report | Publication date (day/month/year) | Patent family member(s) | Publication date (day/month/year) |
|---|---|---|---|
| US 2021/0179581 A1 | 17 June 2021 | WO 2019/028473 A1 | |
| JP 2018-514795 A | 07 June 2018 | US 2018/0149717 A1 | |
| | | WO 2016/188557 A1 | |
| | | EP 3298422 A1 | |
| | | CN 108139454 A | |
| US 2018/0113185 A1 | 26 April 2018 | WO 2016/161278 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014058012 A


### Non-patent literature cited in the description


- **T. VIENNET ; A. VIEGAS ; A. KUEPPER ; S. ARENS ; V. GELEV ; O. PETROV ; T.N. GROSSMANN ; H. HEISE ; M. ETZKORN.** *Angew. Chem. Int. Ed.,* 2016, vol. 55, 10746-10750